# EUROPÄISCHE PATENTANMELDUNG

(11) **EP 1 300 383 A2**
(43) Veröffentlichungstag der Anmeldung: **09.04.2003**
(21) Anmeldenummer: 02021899.6
(22) Anmeldetag: 01.10.2002
(51) Int. Cl.: C07C 41/50, C07C 43/303, C07C 47/198

(54) **Verfahren zur Herstellung von Diacetalen des Glyoxals**

(30) Priorität: 05.10.2001 DE 10149063
(71) Anmelder: BASF AKTIENGESELLSCHAFT, 67056 Ludwigshafen (DE)
(72) Erfinder: Heydrich, Gunnar, 67117 Limburgerhof (DE); Aust, Nicola Christiane, 68199 Mannheim (DE); Pütter, Hermann, 67433 Neustadt (DE); Fischer, Andreas, 67067 Ludwigshafen (DE); Botzem, 67117 Limburgerhof (DE)
(74) Vertreter: Kinzebach, Werner, Dr.

(57) **Zusammenfassung**

Beschrieben wird ein Verfahren zur Herstellung von Diacetalen des Glyoxals durch Umsetzung von 40 bis 75 gew.-%igem, wässrigem Glyoxal mit einwertigen Alkoholen in Gegenwart eines sauren Katalysators, dadurch gekennzeichnet, dass man eine flüssige Mischung, die zu Beginn der Reaktion Alkohol und Glyoxal im Molverhältnis von wenigstens 15:1 sowie Wasser in einer Konzentration von nicht mehr als 8 Gew.-% enthält, solange mit dem sauren Katalysator in Kontakt belässt, bis die Konzentration des gebildeten Diacetals in der Reaktionsmischung wenigstens 70 % der Gleichgewichtskonzentration erreicht hat, ohne dass zuvor mehr als 5 Gew.-% des eingesetzten Alkohols abdestilliert wurden.

## Beschreibung

Die vorliegende Erfindung betrifft ein Verfahren zur Herstellung von Diacetalen des Glyoxals durch Umsetzung von 40 bis 75 gew.-%igem, wässrigem Glyoxal mit einwertigen Alkoholen in Gegenwart eines sauren Katalysators.

Diacetale des Glyoxals, die zum Teil auch als Tetraacetale bezeichnet werden, sind interessante Vorprodukte für die organische Synthese. Die wichtigste Herstellungsmethode für Diacetale des Glyoxals ist die sauer katalysierte Acetalisierung von Glyoxal mit einwertigen Alkoholen R-OH gemäß dem nachfolgenden Schema:

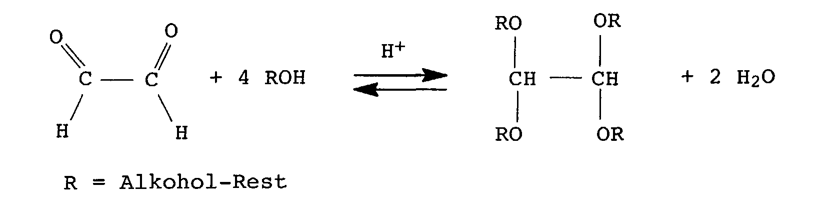

Die säurekatalysierte Acetalisierung von Glyoxal mit einwertigen Alkoholen ist eine komplexe Reaktion, bei der neben dem Monoacetal und dem Diacetal auch eine Vielzahl von Oligomeren und/oder cyclischen Nebenprodukten gebildet werden können (siehe z.B. J.M. Kliegmann et al. in J. Org. Chem. Vol. 38 (1973) S. 556; J. Org. Chem. Vol. 37 (1972) S. 1276ff; A. Stambouli et al., Bull. Soc. Chimique France (1983) II S. 33-40.

Die US 2,360,959 beschreibt die Herstellung von Diacetalen des Glyoxals von Alkoholen, die mit Wasser nicht mischbar sind. Hierzu wird wässriges Glyoxal und eine geringe Menge an saurem Katalysator mit wenigstens 4 Mol des mit Wasser nicht mischbaren einwertigen Alkohols erhitzt, eine azeotrope Mischung aus Wasser und Alkohol abdestilliert und der Alkohol nach Abtrennung des Wassers in die Reaktion zurückgeführt. Die ebenfalls dort beschriebene Umsetzung von Glyoxal mit Methanol erfolgt in analoger Weise, wobei man zur Trennung von Wasser und Alkohol eine zusätzliche, aufwendige fraktionierte Destillation durchführen muß. Dieses Verfahren liefert jedoch im Falle von mit Wasser mischbaren Alkanolen wie Methanol oder Ethanol das entsprechende 1,1,2,2-Tetraalkoxyethan nur in geringen Ausbeuten (38 % für Tetramethoxyethan).

Die GB 559,362 empfiehlt bei der sauer katalysierten Acetalisierung von Glyoxal mit einwertigen Alkoholen die Verwendung eines mit Wasser nicht mischbaren, flüssigen, inerten Lösungsmittels wie Benzol, Toluol, Xylol, Hexan, Dichlorethan oder Isopropylether. Die Verwendung von Schleppmitteln bedeutet jedoch zusätzliche Verfahrenskosten. Zudem sind zumindest die halogenhaltigen Schleppmittel toxikologisch oder ökologisch bedenklich.

F.H. Sangsari et al., Synth. Commun. 18(12) (1988) S. 1343-1348 empfiehlt zur Herstellung von 1,1,2,2-Tetramethoxyethan und 1,1,2,2-Tetraethoxyethan die Acetalisierung in Gegenwart eines Schleppmittels wie Chloroform unter Verwendung einer mit Trockenmittel gefüllten Soxhlett-Apparatur zur Entfernung des bei der Reaktion gebildeten Reaktionswassers.

Die aus dem Stand der Technik bekannten Verfahren zur Herstellung von Diacetalen des Glyoxals haben den Nachteil, dass bei Einsatz von mit Wasser mischbaren Alkoholen wie Methanol oder Ethanol die Ausbeute an Diacetal, d.h. an 1,1,2,2-Tetraalkoxyethan, nur gering ist. Zudem erfordern alle Verfahren zur Entfernung des Reaktionswassers destillative Maßnahmen während der Umsetzung, was mit einem erhöhten energetischen und technischen Aufwand verbunden ist und eine kontinuierliche Durchführung der Reaktion erschwert. Die Verwendung eines Schleppmittels zur Entfernung des Reaktionswassers hat wiederum den Nachteil, dass der ebenfalls mitdestillierte Alkohol und das Schleppmittel aufgetrennt werden müssen.

Der vorliegenden Erfindung liegt somit die Aufgabe zugrunde, ein Verfahren zur Herstellung von Diacetalen des Glyoxals bereitzustellen, das auch bei Einsatz von mit Wasser mischbaren Alkoholen das jeweilige Diacetal in guten Ausbeuten liefert und das in einfacher Weise kontinuierlich ausgestaltet werden kann.

Diese Aufgabe wird überraschenderweise durch ein Verfahren gelöst, bei dem man eine flüssige Mischung, die zu Beginn der Reaktion Alkohol und Glyoxal im Molverhältnis von wenigstens 15:1 sowie Wasser in einer Konzentration von nicht mehr als 8 Gew.-% enthält, solange mit einem sauren Katalysator in Kontakt beläßt, bis sich näherungsweise das Reaktionsgleichgewicht eingestellt hat. Von einer zumindest näherungsweisen Einstellung des Gleichgewichtes wird erfindungsgemäß dann ausgegangen, wenn die Konzentration des gebildeten Diacetals in der Reaktionsmischung wenigstens 70 % der für die jeweilige Zusammensetzung geltenden Gleichgewichtskonzentration an Diacetal erreicht ist.

Demnach betrifft die vorliegende Erfindung ein Verfahren zur Herstellung von Diacetalen des Glyoxals durch Umsetzung von 40 bis 75 gew.-%igem, wässrigem Glyoxal mit einwertigen Alkoholen in Gegenwart eines sauren Katalysators, dadurch gekennzeichnet, dass man eine flüssige Mischung, die zu Beginn der Reaktion Alkohol und Glyoxal im Molverhältnis von wenigstens 15:1 sowie Wasser in einer Konzentration von nicht mehr als 8 Gew.-% enthält, solange mit dem sauren Katalysator in Kontakt beläßt, bis die Konzentration des gebildeten Diacetals in der Reaktionsmischung wenigstens 70 %, vorzugsweise wenigstens 80 %, insbesondere wenigstens 90 % und besonders bevorzugt wenigstens 95 %, der Gleichgewichtskonzentration erreicht hat, ohne dass zuvor mehr als 5 Gew.-% des eingesetzten Alkohols abdestilliert wurden.

Die Konzentration des Wassers in der flüssigen Mischung zu Beginn der Reaktion liegt vorzugsweise im Bereich von 2 bis 8 Gew.-% und beträgt insbesondere nicht mehr als 7 Gew.-%, z.B. 3 bis 7 Gew.-%, und besonders bevorzugt nicht mehr als 6 Gew.-%, z.B. 3 bis 6 Gew.-%, jeweils bezogen auf das Gesamtgewicht der flüssigen Mischung. Das Gesamtgewicht der flüssigen Mischung berechnet sich aus der Summe aller in der Mischung enthaltenen flüssigen und darin gelösten Bestandteile. Es umfaßt nicht die in der Mischung nicht gelösten Bestandteile wie heterogene Katalysatoren. Die Wasserkonzentration kann grundsätzlich auf unterschiedliche Weise eingestellt werden:
a) Beispielsweise kann man ein Glyoxal einsetzen, das einen Gehalt von wenigstens 50 Gew.-% und vorzugsweise wenigstens 60 Gew.-% aufweist. Der Glyoxalgehalt der wässrigen Glyoxal-Lösung wird dabei vorzugsweise einen Wert von 75 Gew.-% nicht überschreiten.
b) Eine zweite Möglichkeit besteht darin, einen großen Überschuß an Alkohol einzusetzen, z.B. mehr als 30 Mol Alkohol, pro Mol Glyoxal, z.B. 30 bis 50 Mol Alkohol/mol Glyoxal in der flüssigen Mischung.
c) Eine dritte Möglichkeit der Einstellung des Wassergehaltes zu Beginn der Reaktion besteht darin, dass man der flüssigen Mischung eine inerte Substanz zusetzt, die in der flüssigen Mischung vollständig löslich und/oder mit dieser vollständig mischbar ist. Sofern eine inerte Substanz verwendet wird, setzt man diese in der Regel in einer Menge von wenigstens 1 Gew.-%, z.B. 1 bis 25 Gew.-%, vorzugsweise 2 bis 20 Gew.-%, ein.

Bevorzugt sind die Maßnahmen a) und c), insbesondere a). Selbstverständlich können die oben genannten Maßnahmen auch miteinander kombiniert werden, vorzugsweise Maßnahme a) mit Maßnahme b) oder Maßnahme a) mit Maßnahme c).

Als inerte Substanz kommen grundsätzlich aprotische organische Lösungsmittel sowie neutrale oder schwach saure Salze in Betracht, welche die Acetalisierung nicht katalysieren. Bevorzugt sind neutrale oder schwach saure Salze. Beispiele für geeignete Salze sind die Halogenide, Sulfate, Nitrate, Monoalkylsulfate, Arylsulfonate und Alkylsulfonate von Metallen der ersten und der zweiten Hauptgruppe z.B. von Na, K, Li oder Mg, von quartären Ammoniumkationen sowie von Eisen(II)- und Eisen(III)-Ionen. Bevorzugte Salze sind die Sulfate, Monoalkylsulfate, Arylsulfonate und Alkylsulfonate der vorgenannten Metalle bzw. der quartären Ammoniumkationen. Als Beispiel für ein Alkylsulfat ist insbesondere Methylsulfat zu nennen. Beispiele für Arylsulfonate sind insbesondere die Phenylsulfonate und die Tolylsulfonate. Ein Beispiel für Alkylsulfonate ist Methylsulfonat.

Beispiele für quartäre Ammoniumkationen sind die Tetrakis-C₁-C₁₀-alkylammoniumkationen wie Methyltriethylammonium und Methyltributylammonium sowie die Phenyl- und Benzyl-tris-C₁-C₄-alkylammoniumkationen wie Benzyltrimethylammonium oder Benzyltriethylammonium.

Beispiele für geeignete Salze, die in der flüssigen Mischung hinreichend löslich sind und die unter den Reaktionsbedingungen inert sind, d.h. die nicht die Acetalisierung katalysieren, sind insbesondere die Tetrakis-C₁-C₁₀-alkylammoniumalkylsulfate wie Methyltriethylammoniummethylsulfat und Methyltributylammoniummethylsulfat.

Sofern als Acetalisierungskatalysator ein heterogener Katalysator, insbesondere ein stark saures Ionentauscherharz verwendet wird, enthält die Reaktionsmischung üblicherweise kein inertes Salz.

Sofern man im erfindungsgemäßen Verfahren wässriges Glyoxal mit einem Glyoxalgehalt von wenigstens 50 Gew.-% und vorzugsweise wenigstens 60 bis 75 Gew.-% verwendet, stellt man dieses durch Aufkonzentrieren von handelsüblichem wässrigem Glyoxal, welches üblicherweise einen Glyoxalgehalt von etwa 40 Gew.-% aufweist, unter vermindertem Druck her. Das Aufkonzentrieren erfolgt vorzugsweise unmittelbar vor der Verwendung des wässrigen Glyoxals, d.h. das aufkonzentrierte wässrige Glyoxal wird nicht länger als länger als 30 min vor seiner Verwendung zwischengelagert. Das Einengen erfolgt vorzugsweise bei einem Druck unterhalb 500 mbar, z.B. 10 bis 500 mbar, vorzugsweise unterhalb 300 mbar und insbesondere im Bereich von 50 bis 300 mbar. Vorzugsweise erfolgt das Aufkonzentrieren des wässrigen Glyoxals bei Temperaturen im Bereich von 40 bis 100 °C, abhängig von dem gewünschten Druck und der Verdampferart.

Vorzugsweise bedient man sich zum Einengen des wässrigen Glyoxals solcher Apparaturen, die ein möglichst schonendes Einengen erlauben, d.h. die beim Einengen eine möglichst geringe thermische Belastung des Destillationsrückstandes zur Folge haben. Geeignete Apparaturen sind grundsätzlich solche, die auf der Verdampfung dünner Filme beruhen, d.h. Dünnschichtverdampfer wie Fallfilmverdampfer, Blasrohrverdampfer, Rotationsdünnschichtverdampfer, z.B. Sambay-Verdampfer oder Luwa-Filmtruder, sowie Zentrifugalverdampfer. Weiterhin geeignet sind Umlaufverdampfer wie insbesondere Zwangsumlaufverdampfer, Zwangsumlaufentspannungsverdampfer, ferner Naturumlaufverdampfer. Als Verdampferbauformen sind sowohl Röhren- als auch Plattenapparate geeignet. Derartige Verdampfer sind dem Fachmann bestens bekannt und beispielsweise beschrieben in R.K. Shah und A.C. Mueller, "Heat Exchange" Kap. 2.2.2.1, Ullmann's Encyclopedia of Industrial Chemistry, 6th ed. on CD-ROM, Wiley VCH.

Geeignet sind insbesondere mehrstufige Ausführungen der vorgenannten Verdampfer, bei denen sowohl gleiche als auch verschiedene Verdampfer miteinander kombiniert werden können. Beispielsweisen kann man zunächst in einem Umlaufverdampfer einen Teil des Wassers entfernen und dann den gewünschten Glyoxalgehalt in einem Dünnschichtverdampfer einstellen. Werden die Stufen bei unterschiedlichen Druckniveaus betrieben, kann man die thermische Energie der Brüden der höheren Druckstufe zum Verdampfen der niedrigeren Druckstufe ausnutzen.

Zum Einengen des wässrigen Glyoxals können weiterhin mehrstufige Trennapparate wie Gegenstromdestillationskolonnen mit Einbauten eingesetzt werden. Weiterhin kann man einen Teil des Wassers in Apparaten zum Verdunsten von Flüssigkeiten entfernen z.B. in Venturi- oder Sprüh-Apparaten oder in Fallfilm-Apparaten, die im Gleich- oder im Gegenstrom betrieben werden können.

Häufig wird man die Brüden, welche Wasser und Glyoxalspuren enthalten, in einem Kondensator niederschlagen. Man kann auch durch sog. Brüdenkompression (Thermische Kompression) mittels eines Brüdenkompressors einen Teil der thermischen Energie der Brüden zum Erwärmen des des wässrigen Glyoxals verwenden und gleichzeitig das Wasser kondensieren.

Geeignet sind weiterhin Stripp-Kolonnen, z.B. solche mit Einbauten, z.B. Füllkörper-, Packungs- oder Bodenkolonnen, oder ohne Einbauten, sog. Sprühkolonnen. Zum Einengen wird man bei diesen Typen das gegebenenfalls vorgewärmte wässrige Glyoxal am Kopf dieser Apparate aufgeben und das Strippgas, vorzugsweise ein Inertgas, welches vorzugsweise vorgewärmt ist, in den unteren Teil der Kolonne einleiten. Hierbei fällt die aufkonzentrierte wässrige Glyoxallösung am Sumpf der Stripp-Kolonne an, während das mit Wasser angereicherte Stripp-Gas am oberen Teil des Apparates austritt. Bei geschlossener Fahrweise kann man die Feuchtbeladung des Strippgases durch Abkühlen in einem Kondensator reduzieren, das so getrocknete Gas wieder Erwärmen und erneut in die Strippkolonne einleiten.

Grundsätzlich können im erfindungsgemäßen Verfahren alle gängigen einwertigen Alkohole eingesetzt werden. Bevorzugt eignen sich Alkohole, deren OH-Gruppe sich an einem primären oder sekundären, vorzugsweise primären aliphatischen C-Atom befindet. Beispiele hierfür sind C₁-C₁₀-Alkanole wie Methanol, Ethanol, n- oder isoPropanol, n-, iso- oder sec-Butanol, n-Hexanol, n-Octanol, 2-Ethylhexan-1-ol, C₅-C₁₀-Cycloalkanole wie Cyclopentanol oder Cyclohexanol, allylische Alkanole wie Allylalkohol, Arylalkanole wie Phenylethanol oder Benzylalkohol.

Erfindungsgemäß bevorzugt sind die mit Wasser mischbaren Alkohole wie Methanol, Ethanol, n- und Isopropanol. Die Herstellung von 1,1,2,2-Tetramethoxyethan durch Acetalisierung von Glyoxal mit Methanol ist eine besonders bevorzugte Ausgestaltung des erfindungsgemäßen Verfahrens.

Das Molverhältnis von Alkohol zu Glyoxal beträgt erfindungsgemäß wenigstens 15:1 und liegt vorzugsweise im Bereich von 15:1 bis 50:1 und insbesondere im Bereich von 18:1 bis 30:1. Höhere Alkoholanteile sind möglich (s.o.), haben jedoch in der Regel einen höheren Arbeitsaufwand zur Folge.

Als saure Katalysatoren kommen sowohl Lewis- als auch Brönstedt-Säuren in Betracht, wie sie bekanntermaßen zur Acetalisierung von Aldehyden verwendet werden können. Als Katalysatoren können sowohl solche, die sich homogen in der Mischung lösen, als auch heterogene Katalysatoren verwendet werden. Beispiele für homogene Katalysatoren sind insbesondere Schwefelsäuren sowie organische Sulfonsäuren wie Methansulfonsäure und p-Toluolsulfonsäure. EbenAls heterogene Katalysatoren kommen beispielsweise Zirkoniumsulfat sowie stark saure, insbesondere sulfonsaure, Ionentauscher in Betracht. Beispiele für geeignete saure Ionentauscher, die insbesondere in makroporöser Form verwendet werden, sind Ionentauscher, die unter den Marken Lewatit®, BayKat® (Bayer AG, Leverkusen), Amberlite® und Amberlyst® (Rohm and Haas) sowie Dowex® (Dow Chemicals) vertrieben werden. Beispiele für handelsübliche stark saure Ionenaustauscher in makroporöser Form sind Lewatit®S 100, Lewatit®K 2431, Lewatit®K 2621, Lewatit®K 2629, BayKat®K 2611, Amberlyst®15, Amberlyst®35 sowie Dowex®50. Die Menge an saurem Katalysator beträgt bei homogener Katalyse in der Regel wenigstens 0,5 Gew.-%, vorzugsweise wenigstens 1 Gew.-% und bei Verwendung eines heterogenen Katalysators in der Regel wenigstens 5 Gew.-%, insbesondere wenigstens 10 Gew.-%, jeweils bezogen auf das Gesamtgewicht der flüssigen Mischung. Höhere Katalysatormengen sind in der Regel nicht nachteilig. Insbesondre bei heterogener Katalyse ist die Katalysatormenge lediglich durch das Flüssigkeitsvolumen begrenzt, wobei die maximale Katalysatormenge einem Katalysatorfestbett entspricht.

In einer bevorzugten Ausgestaltung des erfindungsgemäßen Verfahrens setzt man einen heterogenen Katalysator in Form eines Katalysatorfestbettes ein, durch das die flüssige Mischung, umfassend Alkohol und wässriges Glyoxal, geleitet wird. Die flüssige Mischung kann mehrfach über das Katalysatorfestbett geleitet werden, bis die gewünschte Kontakt- bzw. Verweilzeit erreicht ist. Bevorzugt wird jedoch die Flüssigkeit nur einmal über das Katalysatorbett geleitet, d.h. in geradem Durchgang.

Die zur Acetalisierung erforderlichen Temperaturen liegen in der Regel oberhalb Raumtemperatur und betragen vorzugsweise wenigstens 50°C und liegen insbesondere im Bereich von 50 bis 80°C und besonders bevorzugt im Bereich von 65 bis 75°C. Bei diskontinuierlicher Fahrweise bietet es sich insbesondere an, bei der Siedetemperatur der Reaktionsmischung zu arbeiten. Das erfindungsgemäße Verfahren kann bei Normaldruck, bei vermindertem oder auch bei erhöhtem Druck durchgeführt werden. Vorzugsweise führt man das Verfahren bei Normaldruck oder einem Überdruck von bis zu 5 bar durch.

Im erfindungsgemäßen Verfahren wird die Reaktion des Glyoxals mit dem Alkohol zumindest bis zum annähernden Erreichen des Gleichgewichtszustandes durchgeführt, d.h. soweit, bis die Konzentration des Diacetals in der Reaktionsmischung wenigstens 70, vorzugsweise wenigstens 80 %, insbesondere wenigstens 90% und besonders bevorzugt wenigstens 95 % des Wertes der Gleichgewichtskonzentragleichgewicht einstellt, läßt sich schnell in einem Vorversuch anhand einer Reaktionskinetik ermitteln. Sie beträgt unter den oben angegebenen Reaktionsbedingungen in der Regel wenigstens 2 und vorzugsweise wenigstens 3 Stunden. Häufig ist das Gleichgewicht bereits nach 10 Stunden erreicht. Selbstverständlich sind auch längere Reaktionszeiten, z.B. bis 36 Stunden und vorzugsweise bis 24 Stunden möglich. Vorzugsweise beträgt die Reaktionszeit 3 bis 8 Stunden.

Nach Erreichen des Reaktionsgleichgewichtes wird der saure Katalysator im Falle der homogenen Katalyse durch Neutralisation mit einer geeigneten Base wie Alkali- oder Erdalkalihydroxiden wie NaOH, KOH oder Ca(OH)₂, vorzugsweise durch Neutralisation mit Alkalicarbonaten wie Natrium- oder Kaliumcarbonat oder mit Alkalihydrogencarbonaten wie Natrium- oder Kaliumhydrogencarbonat im Reaktionsgemisch deaktiviert. Im Falle der heterogenen Katalyse wird der Katalysator vom Reaktionsgemisch separiert, z.B. durch Filtration oder bei Verwendung eines Katalysatorfestbettes durch Abkoppeln des Stoffstroms vom Katalysatorbett.

Nach Deaktivierung bzw. Abtrennung des sauren Katalysators wird die erhaltene flüssige Mischung in üblicher Weise destillativ aufgearbeitet. In der Regel wird zunächst der überschüssige Alkohol destillativ entfernt. Der abdestillierte Alkohol wird vorzugsweise im nächsten Reaktionsansatz wieder eingesetzt oder bei kontinuierlicher Fahrweise dem Reaktor wieder zugeführt. Die Gewinnung des Diacetals und seine Abtrennung von ebenfalls gebildetem Monoacetal und gegebenenfalls höhermolekularen Nebenprodukten kann grundsätzlich auf die in der DE-A 196 51 325 beschriebenen Weise erfolgen. Dabei geht man in der Regel so vor, dass man den nach destillativem Entfernen des Alkohols verbleibenden Destillationsrückstand unter Zusatz von Wasser erneut destilliert, wobei das gewünschte Glyoxaldiacetal und der überwiegende Teil des Wassers als Homoazeotrop abdestilliert. Es hat sich bewährt, einen Teil des zugesetzten Wassers in Form des beim Einengen des wässrigen Glyoxals gewonnenen Kondensats einzusetzen, da auf diese Weise das im Kondensat enthaltene Glyoxal zurückgewonnen werden kann. Der Wasserzusatz wird vorzugsweise so bemessen, dass die Gesamtmenge aus Wasserzusatz und dem in Reaktionsmischung bereits vorhandenen Wasser gerade der Wassermenge entspricht, die für das Homoazetrop Wasser/Glyoxaldiacetal erforderlich ist. Die erforderliche Menge kann der Fachmann leicht anhand der Konzentrationen von Glyoxal und Wasser im Reaktionsgemisch ermitteln. Der Destillationsrückstand, der im Wesentlichen das Monoacetal und nicht umgesetztes Glyoxal, gegebenenfalls Reste an Diacetal, geringen Mengen an Wasser und gegebenenfalls höhere oligomere Nebenprodukte enthält, kann, erforderlichenfalls nach Entwässern, ebenfalls in die Reaktion zurückgeführt werden.

Zur Gewinnung des Glyoxaldiacetals kann das bei der Destillation anfallende Wasser/Glyoxaldiacetal-Homoazeotrop in üblicher Weise entwässert werden, beispielsweise durch azeotrope Destillation in Gegenwart von den oben genannten Schleppmitteln, insbesondere Hexan, Cyclohexan, Heptan, Octan, Toluol oder Xylol. Diese Vorgehensweise hat insbesondere den Vorteil, dass eine aufwendige Abtrennung von Alkohol und Schleppmittel, wie es bei den Verfahren des Standes der Technik erforderlich ist, vermieden wird.

In einer bevorzugten Ausführungsform der vorliegenden Erfindung führt man die Umsetzung von Glyoxal mit dem einwertigen Alkohol kontinuierlich an einem heterogenen, stark sauren Katalysator durch. Hierbei geht man in der Regel so vor, dass man eine flüssige Mischung, die wässriges Glyoxal und den einwertigen Alkohol sowie nicht mehr als 8 Gew.-%, vorzugsweise 2 bis 8 Gew.-% und insbesondere 3 bis 7 Gew.-% Wasser enthält, kontinuierlich in einen Reaktor einspeist, der einen heterogenen, sauren Katalysator enthält, und dem Reaktor kontinuierlich eine flüssige Reaktionsmischung entnimmt. Die Verweilzeit im Reaktor wird erfindungsgemäß so gewählt, dass die Konzentration des Diacetals in der ausgeschleusten, flüssigen Reaktionsmischung wenigstens 70 %, vorzugsweise wenigstens 80 %, insbesondere wenigstens 90 % und besonders bevorzugt wenigstens 95 % der Gleichgewichtskonzentration beträgt.

Die mittlere Verweilzeit der Reaktionsmischung im Reaktor liegt in der Regel unter den obigen Reaktionsbedingungen im Bereich von 2 Stunden bis 10 Stunden und vorzugsweise im Bereich von 3 Stunden bis 8 Stunden.

Hinsichtlich der Art des Katalysators, des Drucks und der Temperatur gelten grundsätzlich die oben gemachten Angaben. Bevorzugt arbeitet man jedoch bei einem geringen Überdruck, z.B. 1,1 bis 5 bar. Die Aufarbeitung des flüssigen Reaktoraustrages kann ebenfalls in der obigen Weise erfolgen.

Die kontinuierliche Durchführung des erfindungsgemäßen Verfahrens kann in den für eine kontinuierliche Umsetzung von Flüssigkeiten an heterogenen Katalysatoren üblichen Reaktoren erfolgen, beispielsweise in kontinuierlich betriebenen Rührkesseln, Kolonnen oder vorzugsweise in Reaktoren mit rohrförmiger Reaktorgeometrie (Rohrreaktoren). Bevorzugt werden dabei solche Reaktoren, in denen der heterogene Katalysator in Form eines oder mehrerer Festbetten angeordnet ist. Hierzu zählen Rohrreaktoren und Kolonnen.

Erfindungsgemäß bevorzugt sind Rohrreaktoren, da hier eine besonders rasche Einstellung des Acetalisierungsgleichgewichtes erreicht wird. Rohrreaktoren umfassen hier und im Folgenden sowohl Schachtreaktoren (einzelne Reaktionsrohre) als auch Rohrbündelapparate. Die Rohrreaktoren können horizontal angeordnet sein und sind bevorzugt vertikal angeordnet. Bei vertikaler Anordnung kann die flüssige Reaktionsmischung sowohl aufwärts als auch abwärts durch den Reaktor geleitet werden.

In den rohrförmigen Reaktoren ist der heterogene Katalysator in der Regel in Form einer oder mehrerer Schüttungen angeordnet, wobei die Reaktoren in der Regel mit Vorrichtungen versehen sind, die ein Austragen des Katalysators während des Betriebes weitestgehend vermeiden. Auch die Verwendung mehrerer verschiedener Katalysatoren in sogenannten strukturierten Schüttungen ist möglich.

Weiterhin können die Rohrreaktoren an ein oder mehreren Stellen mit Anbauten versehen sein, durch die Wärme zu- oder abgeführt werden kann. Beispiele für derartige Einbauten sind Rohrwendeln, horizontal oder vertikal angeordnete Rohre oder Platten. Durch diese Einbauten können gezielt flüssige oder dampfförmige Wärmeträger geleitet werden. Weiterhin können Kondensations- und Verdampfungs-Vorgänge im Reaktor zur Wärmeab- bzw. -zufuhr in bekannter Weise genutzt werden.

Bei der kontinuierlichen Ausgestaltung des erfindungsgemäßen Verfahrens hat es sich als vorteilhaft erwiesen, wenn man Alkohol und wässriges Glyoxal nicht als separate Ströme sondern in Form der oben beschiebenen flüssigen Mischung in den Reaktor einspeist. Die flüssige Mischung kann beispielsweise in einem Vorratsgefäß durch Vermischen von wässrigem Glyoxal der gewünschten Konzentration und dem Alkohol sowie gegebenenfalls dem rückgeführten Alkohol und gegebenenfalls dem rückgeführten Acetal herstellen und anschließend die so erhaltene Mischung in den Reaktor einleiten. Man kann jedoch so vorgehen, dass man die einzelnen Stoffströme nacheinander oder gleichzeitig über Vorrichtungen zum Vermischen von Flüssigkeiten leitet und in den Reaktor einspeist. Geeignet sind grundsätzlich alle bekannten Vorrichtungen zum kontinuierlichen Mischen von Flüssigkeiten wie Jet-Mischer, statische Mischer und dynamische Mischer. Beispiele für derartige Mischer sind dem Fachmann bekannt, z.B. aus H.J. Hensler, "Continuous Mixing of Fluids" in Ullmann's Encyclopedia of Industrial Chemistry, 5^{th} ed. on CD-Rom, Wiley-VCH, Weinheim.

In einer bevorzugten Ausführungsform des kontinuierlichen Verfahrens stellt man zuerst in der oben beschriebenen Weise 60 bis 75 gew.-%iges wässriges Glyoxal durch Einengen einer konventionellen wässrigen Glyoxal-Lösung bei vermindertem Druck her und überführt diese unmittelbar im Anschluß daran gemeinsam mit dem Alkohol, der sowohl frischen Alkohol als auch gegebenenfalls rückgeführten Alkohol umfasst, und gegebenenfalls zusammen mit rückgeführtem Mono- und gegebenenfalls Diacetal in den Reaktor.

Weiterhin hat es sich bewährt, den bei der Aufarbeitung abgetrennten Alkohol in die Umsetzung zurückzuführen, so dass zumindest ein Teil des Alkohols in der Mischung, welche in den Reaktor eingespeist wird, aus dem rückgeführten Alkohol resultiert. Dies kann zur Folge haben, dass die in den Reaktor eingespeiste Mischung geringe Anteile an Mono- und Diacetal enthält. Auch ist es möglich, die bei der Aufarbeitung anfallenden höher siedenden Fraktionen, die neben dem Glyoxalmonoacetal gegebenenfalls noch Reste an Glyoxaldiacetal und höhersiedende Oligomere enthalten, in die kontinuierliche Umsetzung zurückzuführen, z.B. zusammen mit dem rückgeführten Alkohol. Die Stoffströme werden selbstverständlich so gewählt, dass das gewünschte Alkohol/Glyoxal-Verhältnis und der Wassergehalt in den erfindungsgemäßen Bereichen liegt.

Das erfindungsgemäße Verfahren liefert die Diacetale des Glyoxals in hohen Ausbeuten. Ein Abdestillieren des bei der Reaktion gebildeten Wassers ist überraschenderweise nicht zur Erreichung der hohen Ausbeuten erforderlich. Das erfindungsgemäße Verfahren kann daher in einfacher Weise kontinuierlich ausgestaltet werden. Eine sowohl energetisch als auch apparativ aufwendige Trennung von Wasser und Alkohol während der Umsetzung ist nicht erforderlich.

Die nun folgenden Beispiele sollen die Erfindung verdeutlichen, ohne sie jedoch einzuschränken.

### Beispiel 1:

58 g einer 40 gew.-%igen wässrigen Glyoxallösung wurden am Rotationsverdampfer unter vermindertem Druck (10 mbar) eingeengt. Die erhaltenen 33,1 g wässriges, 70 %iges Glyoxal und 256 g Methanol wurden mit 72 g methanolfeuchtem, stark saurem Ionentauscher (Amberlyst®15) 2 h zum Rückfluss erhitzt. Der Wassergehalt der flüssigen Bestandteile betrug 2,7 Gew.-%. Die anschließende Analyse per Gaschromatographie ergab einen Gehalt der Reaktionslösung an 1,1,2,2-Tetramethoxyethan von 13,6 Gew.-% und an Glyoxaldimethylacetal von 2,48 Gew.-%. Dies entspricht einer Rohausbeute von 65,3 % 1,1,2,2-Tetramethoxyethan und von 17,2 % Glyoxaldimethylacetal, bezogen auf eingesetztes Glyoxal.

### Beispiel 2:

Ein Rohrreaktor mit einem Reaktionsvolumen von 0,3 l und einer effektiven Länge von 22 cm wurde mit 219 g Ionentauscher Amberlyst®5 befüllt. Eine Lösung bestehend aus 70 gew.-%igem, wässrigem Glyoxal, hergestellt wie in Beispiel 1, und Methanol in einem Molverhältnis von Methanol:Glyoxal von 20 (Glyoxal-Gehalt 8 %, Wassergehalt 3,4 Gew.-%) wurde mit 60 ml/h kontinuierlich in geradem Durchgang bei 60°C über das Katalysatorbett gepumpt. Nach einer Verweilzeit von 5 h enthielt der Austrag 14,5 Gew.-% Tetramethoxyethan und 3,5 Gew.-% Glyoxaldimethylacetal. Dies entspricht 70 % Ausbeute an Tetramethoxyethan und 24,7 % Ausbeute an Glyoxaldimethylacetal, bezogen auf Glyoxal.

### Beispiel 3:

58 g 40 gew.-%ige, wässrige Glyoxallösung wurde analog Beispiel 1 unter vermindertem Druck eingeengt und anschließend umgesetzt. Die erhaltenen 33,1 g 70 %iges, wässriges Glyoxal wurden in 256 g Methanol unter Zusatz von 10,9 g Methansulfonsäure 24 h unter Rückfluss erhitzt (Molverhältnis Methanol:Glyoxal 20:1, Wassergehalt zu Beginn 3,3 Gew.-%). Die anschließende Analyse per Gaschromatographie ergab einen Gehalt der Reaktionslösung von 15,0 Gew.-% 1,1,2,2-Tetramethoxyethan und von 2,55 Gew.-% Glyoxaldimethylacetal. Dies entspricht einer Rohausbeute von 67,3 % 1,1,2,2-Tetramethoxyethan und von 16,5 % Glyoxaldimethylacetal, bezogen auf Glyoxal.

### Beispiel 4:

58 g 40 %ige, wässrige Glyoxallösung wurde analog Beispiel 1 unter vermindertem Druck eingeengt und anschließend umgesetzt. Die erhaltenen 33,1 g 70 %iges, wässriges Glyoxal wurden in 238,9 g Methanol unter Zusatz von 10,8 g Methansulfonsäure und 50,7 g Methyltriethylammoniummethylsulfat (60 %ig in Methanol) auf die in Beispiel 3 beschriebene Weise umgesetzt (Wassergehalt 3,0 Gew.-%, Molverhältnis Methanol:Glyoxal = 20:1). Man erhielt nach 24 h unter Rückfluss eine Lösung mit 13,91 Gew.-% 1,1,2,2-Tetramethoxyethan und 2,69 Gew.-% Glyoxaldimethylacetal. Dies entspricht Rohausbeuten von 73 % 1,1,2,2-Tetramethoxyethan und 20,4 % Glyoxaldimethylacetal, bezogen auf Glyoxal.

### Beispiel 5:

58 g 40 %ige Glyoxallösung wurde analog Beispiel 1 unter vermindertem Druck eingeengt und anschließend umgesetzt. Die erhaltenen 34,2 g 70 %iges, wässriges Glyoxal wurden in 256 g Methanol unter Zusatz von 10,8 g Methansulfonsäure und 12,4 g Methyltributylammoniummethylsulfat 24 h zum Rückfluss erhitzt (Wassergehalt 3,3 Gew.-%, Molverhältnis Methanol:Glyoxal = 20:1). Die erhaltene Lösung hatte einen Gehalt von 13,8 Gew.-% 1,1,2,2-Tetramethoxyethan und von 2,24 % Glyoxaldimethylacetal, bestimmt mittels Gaschromatographie. Man erhält als Rohausbeute 70,9 % 1,1,2,2-Tetramethoxyethan und 16,6 % Glyoxaldimethylacetal, bezogen auf Glyoxal.

### Beispiel 6 (Vergleich)

Ein Rohrreaktor (Länge 1 m, Durchmesser 3,5 cm, Innenvolumen etwa 950 ml) wurde vollständig mit dem Ionentauscher Lewatit®K2431 befüllt und mit Methanol geflutet. In den Reaktor wurde nunmehr eine Mischung aus 40 gew.-%igem Glyoxal und Methanol mit einem Molverhältnis von Methanol:Glyoxal von 20:1 und einem Wassergehalt von 11,1 Gew.-% mit einer Zufuhrrate von 175 ml/h kontinuierlich bei 65°C über das Katalysatorbett gepumpt. Nach 28 h enthielt der Reaktoraustrag 9 Gew.-% Tetramethoxyethan, 5,2 Gew.-% Glyoxaldiacetal und 1 Gew.-% Glyoxal. Dies entspricht einer Ausbeute an Tetramethoxyethan von 44 %, bezogen auf Glyoxal.

### Beispiel 7

In einem kontinuierlich betriebenen Fallfilmverdampfer wurden 1678 g einer handelsüblichen 40 gew.-%igen wässrigen Glyoxallösung bei 100 mbar zu etwa 1032 g einer etwa 65 gew.-%igen Lösung eingeengt. Die so erhaltene Glyoxal-Lösung wurde unmittelbar danach mit 5502 g Methanol verdünnt (Molverhältnis Methanol:Glyoxal etwa 15:1, Wassergehalt 5,6 Gew.-%). Diese Lösung wurde mit einer Zufuhrrate von 156 ml/h durch einen bei 65°C temperierten Rohrreaktor (Länge 1 m, Durchmesser 3,5 cm), der mit 950 ml Lewatit-®K2629 befüllt war, gepumpt. Nach 28 h kontinuierlichem Betrieb enthält der Reaktoraustrag 14 Gew.-% Tetramethoxyethan, 5,1 Gew.-% Glyoxalmonoacetal und 0,6 Gew.-% Glyoxal. Dies entspricht einer Ausbeute an Tetramethoxyethan von 53 %, bezogen auf Glyoxal.

### Beispiel 8 (Vergleich)

In einem kontinuierlich betriebenem Umlaufverdampfer wurden 1273,4 g einer handelsüblichen, 40 gew.-%igen Glyoxallösung bei einem Druck von 100 mbar zu 783,6 g einer 65 gew.-%igen, wässrigen Glyoxallösung aufkonzentriert. Die Lösung wird unmittelbar im Anschluß daran mit 2809,5 g Methanol verdünnt (Molverhältnis Methanol:Glyoxal etwa 10:1, Wassergehalt 7,7 Gew.-%). Diese Lösung wurde mit einer Zufuhrrate von 160 ml/h durch den im Beispiel 7 beschriebenen, auf 65°C temperierten Rohrreaktor gepumpt. Nach einer Betriebsdauer von 28 h enthielt der Reaktoraustrag 15,1 Gew.-% Tetramethoxyethan, 9,1 Gew.-% Glyoxalmonoacetal und 1,4 Gew.-% Glyoxal. Dies entspricht einer Ausbeute an Tetramethoxyethan, bezogen auf Glyoxal von 41 %.

### Beispiel 9

In einem kontinuierlich betriebenen Dünnschichtverdampfer wurden 1351 g einer 40 gew.-%igen, wässrigen Glyoxallösung bei 200 mbar zu 831,5 g einer 65 gew.-%igen Lösung eingeengt. Die Lösung wurde unmittelbar im Anschluß mit insgesamt 4472 g Methanol verdünnt (Molverhältnis Methanol:Glyoxal von 15:1 und Wassergehalt 5,6 Gew.-%). Die so erhaltene Lösung wurde mit einer Zufuhrrate von 199 ml/h bei Umgebungsdruck durch den in Beispiel 7 beschriebenen, auf 64°C temperierten Reaktor gepumpt. Der Reaktoraustrag enthielt nach einer Betriebsdauer von 28 h unter anderem 14,4 Gew.-% Tetramethoxyethan, 5,7 Gew.-% Glyoxalmonoacetal und 0,5 Gew.-% Glyoxal. Dies entspricht einer Ausbeute an Tetramethoxyethan von 55 %, bezogen auf eingesetztes Glyoxal.

In allen Beispielen wurde die Umsetzung bis zum Erreichen des Gleichgewichts geführt. Die Konzentration des Tetramethoxyethans in den Reaktionsmischungen lag in allen Fällen oberhalb des 90 %-Wertes der Gleichgewichtskonzentration.

## Patentansprüche

1. Verfahren zur Herstellung von Diacetalen des Glyoxals durch Umsetzung von 40 bis 75 gew.-%igem, wässrigem Glyoxal mit einwertigen Alkoholen in Gegenwart eines sauren Katalysators, **dadurch gekennzeichnet, dass** man eine flüssige Mischung, die zu Beginn der Reaktion Alkohol und Glyoxal im Molverhältnis von wenigstens 15:1 sowie Wasser in einer Konzentration von nicht mehr als 8 Gew.-% enthält, solange mit dem sauren Katalysator in Kontakt belässt, bis die Konzentration des gebildeten Diacetals in der Reaktionsmischung wenigstens 70 % der Gleichgewichtskonzentration erreicht hat, ohne dass zuvor mehr als 5 Gew.-% des eingesetzten Alkohols abdestilliert wurden.

2. Verfahren nach Anspruch 1, **dadurch gekennzeichnet, dass** man ein 60 bis 75 gew.-%iges, wässriges Glyoxal einsetzt.

3. Verfahren nach Anspruch 1 oder 2, **dadurch gekennzeichnet, dass** das Molverhältnis von Alkohol zu Glyoxal zu Beginn der Reaktion im Bereich von 15:1 bis 30:1 liegt.

4. Verfahren nach einem der vorhergehenden Ansprüche, **dadurch gekennzeichnet, dass** der Alkohol ausgewählt ist unter mit Wasser vollständig mischbaren Alkoholen.

5. Verfahren nach Anspruch 4, **dadurch gekennzeichnet, dass** der Alkohol Methanol ist.

6. Verfahren nach einem der vorhergehenden Ansprüche, **dadurch gekennzeichnet, dass** der Katalysator ausgewählt ist unter Schwefelsäure, Schwefelsäurehalbestern, organischen Sulfonsäuren und Sulfonsäuren-Ionenaustauscherharzen.

7. Verfahren nach einem der vorhergehenden Ansprüche, **dadurch gekennzeichnet, dass** man die Reaktion bei einer Temperatur oberhalb 50 °C durchführt.

8. Verfahren nach einem der vorhergehenden Ansprüche, **dadurch gekennzeichnet, dass** man die Umsetzung an einem KatalysatorFestbett durchführt.

9. Verfahren nach einem der Ansprüche 1 bis 7, **dadurch gekennzeichnet, dass** die flüssige Mischung zusätzlich wenigstens ein neutrales oder schwach saures Salz in einer Menge von wenigstens 1 Gew.-%, bezogen auf die Mischung, in gelöster Form enthält.

10. Verfahren nach Anspruch 9, **dadurch gekennzeichnet, dass** das Salz ausgewählt ist unter den Sulfaten, Monoalkylsulfaten, Arylsulfonaten und Alkylsulfonaten von Metallen der ersten und zweiten Hauptgruppe, von quartären Ammoniumkationen sowie von Fe(II)- und Fe(III)-ionen.

11. Verfahren nach einem der Ansprüche 1 bis 8, **dadurch gekennzeichnet, dass** die Umsetzung von Glyoxal mit dem einwertigen Alkohol kontinuierlich an einem heterogenen, sauren Katalysator erfolgt.

12. Verfahren nach Anspruch 11, **dadurch gekennzeichnet, dass** man eine flüssige Mischung aus 40 bis 75 gew.-%igem, wässrigem Glyoxal und dem einwertigen Alkohol, die nicht mehr als 8 Gew.-% Wasser enthält, kontinuierlich in einen Reaktor einspeist, der einen heterogenen, sauren Katalysator enthält, und dem Reaktor kontinuierlich eine flüssige Reaktionsmischung entnimmt, in der die Konzentration des Diacetals wenigstens 80 % der Gleichgewichtskonzentration beträgt.

13. Verfahren nach Anspruch 12, **dadurch gekennzeichnet, dass** man zunächst ein 60 bis 75 gew.-%iges, wässriges Glyoxal durch Einengen einer konventionellen wässrigen Glyoxal-Lösung bei vermindertem Druck herstellt und unmittelbar im Anschluss daran gemeinsam mit dem Alkohol in den Reaktor einspeist.

14. Verfahren nach einem der Ansprüche 12 oder 13, **dadurch gekennzeichnet, dass** der Reaktor eine rohrförmige Geometrie aufweist.

15. Verfahren nach einem der Ansprüche 12 bis 14, **dadurch gekennzeichnet, dass** die Mischung, die in den Reaktor einspeist wird, neben Alkohol, Glyoxal und Wasser noch das Mono- und gegebenenfalls das Diacetal des Glyoxals enthält.
